# EUROPEAN PATENT APPLICATION

(11) **EP 4 840 411 A1**
(43) Date of publication of application: **18.11.2026**
(21) Application number: 25738598.9
(22) Date of filing: 11.01.2025
(51) Int. Cl.: A61B 17/32, A61B 17/16

(54) **PLANING CUTTER**

(30) Priority: 11.01.2024 CN 202420067393 U; 11.01.2024 CN 202420067397 U; 01.02.2024 CN 202420250130 U
(71) Applicant: Guizhou Zirui Technology Co., Ltd, Zunyi, Guizhou 563200 (CN)
(72) Inventor: LI, Hongyuan, Zunyi, Guizhou 563200 (CN); LIU, Changfeng, Zunyi, Guizhou 563200 (CN); LI, Huihua, Zunyi, Guizhou 563200 (CN)
(74) Representative: Metida
(86) International application number: PCT/CN2025/071932
(87) International publication number: WO 2025/149071

(57) **Abstract**

The present invention provides a planing cutter comprising a cutter tube assembly that includes an inner cutter tube and an outer cutter tube, wherein the inner cutter tube is rotatable relative to the outer cutter tube. The present application integrates the cutter tube assembly, the power assembly, the negative pressure suction assembly, and the water injection assembly into a housing to form an integral structure that dispenses with an additional handle, thereby facilitating cleaning and maintenance. The power assembly drivingly rotates the cutter tube assembly to perform planing at a target site of a patient; the negative pressure suction assembly aspirates fragmented tissue produced during planing; and the water injection assembly infuses fluid to the target site. Multiple functions are thus integrated into a single integral planing cutter, enabling adaptation to a wider range of operating scenarios and providing high applicability. By forming the contact surface between the lateral mounting portion of the mounting base and the motor as a concave arcuate surface, the contact area between the motor and the lateral mounting portion is increased, thereby enhancing the cooling effect for the motor.

## Description

### TECHNICAL FIELD

The present invention relates to the technical field of medical devices, and more particularly, to a planing cutter.

### BACKGROUND

In spinal surgery, orthopedic surgery, and neurosurgery, rotary planing and grinding are performed on diseased bone tissue to form internal passageways. In conventional medical planers, an outer cutter tube is typically fixed to a drive handle, which drives an inner cutter tube to spin at high frequency about its own axis. Rotation of the inner cutter tube relative to the outer cutter tube accomplishes resection of cartilage tissue.

Chinese Patent Application Publication No. CN116269661A discloses a soft tissue planing cutter assembly including a planer assembly, a speed reducer assembly, a housing, a suction device, and an interface portion. The planer assembly includes a hollow outer cutter assembly and an inner cutter assembly. The suction device includes a negative pressure suction tube communicating with a cavity of the inner cutter assembly. The soft tissue planing cutter assembly effectively protects the motor by separating the planer assembly from the drive unit and rerouting the negative pressure suction path, but suffers from poor cooling performance and cannot actively infuse fluid to the working site.

Chinese Patent Application Publication No. CN116370036A discloses a soft tissue planing cutter including a planer assembly, a speed reducer assembly, a housing, a motor, and a suction device. The planer assembly includes a hollow outer cutter assembly and an inner cutter assembly, with the inner cutter assembly nested in the outer cutter assembly. The speed reducer assembly is fixed in the housing and includes a speed reducer housing and a transmission gear set. The other end of the outer cutter assembly is connected to the speed reducer housing, and the transmission gear set is connected to the other end of the inner cutter assembly and the output end of the motor, respectively. The suction device includes a suction valve and a suction tube. The speed reducer assembly drives the planer assembly for reciprocal rotation and aspirates soft tissue via the suction device. An adjustment device regulates suction operation for better adaptation to surgical conditions. However, the structure suffers from poor cooling performance and cannot actively infuse fluid to the working site.

Chinese Patent Application Publication No. CN221205577U discloses a planer handle including a handle body with an integrated motor and a harness male connector connected and communicated with the handle body. A cutter identification device is disposed at one end of the handle, and a buckle at the other end. A water tube groove is formed between the cutter identification device and the buckle. The cutter identification device facilitates identification of cutter information and improves operational efficiency; the buckle enables convenient connection to a housing holding a cutter; and the water tube groove provides heat dissipation for the handle. The structure is simple, compact, and easy to use. However, the split-type structure is inconvenient to clean and provides poor cooling performance.

Chinese Patent Application Publication No. CN221469949U discloses a planer cutter head assembly and a planing cutter. The cutter head assembly includes an outer cutter head and an inner cutter head. The outer cutter head defines an outer planing window, and the inner cutter head defines an inner planing window. The outer planing window is formed with outer planing teeth, and the inner planing window is formed with inner planing teeth. The inner planing teeth include a first inner edge, a first outer edge, and a first tip intersection line, where an inner vertex of the first tip intersection line is lower than an outer vertex thereof. The outer planing teeth include a second inner edge, a second outer edge, and a second tip intersection line, where an inner vertex of the second tip intersection line is higher than an outer vertex thereof. The planing cutter further includes an inner tube, an outer tube, and a handle. A power mechanism for driving the inner tube and a front adapter sleeve are disposed in the handle. The outer tube is fixedly connected to the front adapter sleeve. The inner tube is in fixed communication with the inner cutter head, and the outer tube is in fixed communication with the outer cutter head. The cutter head assembly facilitates tissue cutting and improves surgical efficiency, but suffers from poor cooling performance.

Accordingly, there exists an urgent market need for an integrated planing cutter having superior cooling performance and capable of actively infusing fluid to a surgical site.

### SUMMARY

In view of the shortcomings in the prior art, an object of the present invention is to provide an improved planing cutter.

A planing cutter according to the present invention comprises a cutter tube assembly including an inner cutter tube and an outer cutter tube, wherein the inner cutter tube is rotatable relative to the outer cutter tube.

Preferably, the planing cutter further comprises a housing, a power assembly, and a negative pressure suction assembly.

The power assembly is disposed in the housing. One end of the cutter tube assembly extends into the housing, the outer cutter tube is fixedly connected to the housing, and the inner cutter tube is drivingly connected to an output end of the power assembly.

One end of the negative pressure suction assembly extends into the housing and communicates with a cavity of the inner cutter tube, and the other end of the negative pressure suction assembly extends outwardly from the housing.

Preferably, the planing cutter further comprises a water injection assembly. An end of the cutter tube assembly remote from the housing is formed with a water outlet. A water injection gap is defined between an inner wall of the outer cutter tube and an outer wall of the inner cutter tube, the water injection gap being in communication with the water outlet. One end of the water injection assembly extends into the housing and communicates with the water injection gap between the outer cutter tube and the inner cutter tube, and the other end of the water injection assembly extends outwardly from the housing.

Preferably, the power assembly comprises a mounting base, a motor, and a gear transmission structure. The mounting base is fixedly mounted in the housing. The motor is seated on a lateral mounting portion of the mounting base. An output end of the motor abuts a vertical mounting portion of the mounting base, and an output shaft of the motor extends through the vertical mounting portion of the mounting base.

The gear transmission structure includes a driving gear, a dual transmission gear, and a driven gear. The driving gear is coaxially fixed to the output shaft of the motor. The dual transmission gear is rotatably mounted on the vertical mounting portion of the mounting base via a rotating shaft. The driven gear is drivingly connected to the inner cutter tube. The dual transmission gear drivingly connects the driving gear and the driven gear.

Preferably, the negative pressure suction assembly comprises a negative pressure suction tube and a regulating valve. An end of the inner cutter tube extending into the housing extends into the lateral mounting portion of the mounting base. One end of the negative pressure suction tube extends into the lateral mounting portion of the mounting base, and the other end of the negative pressure suction tube extends outwardly from the housing.

The regulating valve is rotatably disposed on the lateral mounting portion of the mounting base and configured to connect or disconnect the negative pressure suction tube and the inner cutter tube. A regulating portion of the regulating valve is located outside the housing.

Seals are disposed at a connection between the inner cutter tube and the lateral mounting portion of the mounting base and at a rotational connection between the regulating valve and the lateral mounting portion of the mounting base.

Preferably, a limiting tube is sleeved on the negative pressure suction tube. One end of the limiting tube abuts a rear end surface of the lateral mounting portion of the mounting base, and the other end of the limiting tube abuts a reinforcing rib inside the housing.

Preferably, a contact surface between the lateral mounting portion of the mounting base and the motor is a concave arcuate surface.

Preferably, the outer cutter tube is fixedly connected to a front end of the lateral mounting portion of the mounting base via a fixed connection sleeve.

The water injection assembly comprises a water injection tube. One end of the water injection tube is disposed inside the housing, and the other end of the water injection tube extends outwardly from the housing. The fixed connection sleeve is formed with a water injection connection hole. An end of the water injection tube inside the housing communicates with the water injection connection hole. The water injection connection hole communicates with the water injection gap between the outer cutter tube and the inner cutter tube.

A seal and a bearing are disposed between an end of the fixed connection sleeve proximate to the mounting base and the inner cutter tube.

A drill bit structure for planing according to the present invention comprises a drill bit coaxially fixed to one end of the inner cutter tube, the drill bit being rotatable relative to the outer cutter tube, wherein an inner bore of the drill bit is in communication with an inner passage of the inner cutter tube.

The drill bit is formed with radial suction bores evenly distributed along a circumference of the drill bit. Each of the radial suction bores communicates with the inner bore of the drill bit.

A planing drill bit assembly according to the present invention comprises a cutter tube assembly including an inner cutter tube and an outer cutter tube, wherein the inner cutter tube is rotatable relative to the outer cutter tube.

The outer cutter tube includes a double-layer wall. A water injection gap is defined between the double-layer wall of the outer cutter tube. A drill bit connection cylinder is formed with a water outlet hole in communication with the water injection gap between the double-layer wall.

A rotating drill bit is formed with a suction bore in communication with an inner cavity of the inner cutter tube.

Preferably, the planing drill bit assembly further comprises a rotating drill bit, a drill bit connection cylinder, and a power adapter sleeve. One end of the drill bit connection cylinder extends into the outer cutter tube and is fixedly connected thereto. One end of the rotating drill bit extends through an interior of the drill bit connection cylinder and into the inner cutter tube. The rotating drill bit is coaxially fixed to the inner cutter tube via the power adapter sleeve. The rotating drill bit is rotatably engaged with the drill bit connection cylinder.

The power adapter sleeve abuts and limits one end of the drill bit connection cylinder. The other end of the rotating drill bit is formed with an abutment surface that abuts and limits the other end of the drill bit connection cylinder.

A planing cutter head for a planing cutter according to the present invention comprises: an outer planing window formed at one end of the outer cutter tube; outer planing teeth formed on the outer planing window, the outer planing teeth including a plurality of continuous tooth portions, each tooth portion of the outer planing teeth being inclined outwardly. One end of the inner cutter tube is formed with an inner planing window. The inner planing window is formed with inner planing teeth, the inner planing teeth including a plurality of continuous tooth portions, each tooth portion of the inner planing teeth being inclined inwardly.

A planing cutter head for a planing cutter according to the present invention comprises an outer planing window form at one end of the outer cutter tube . A continuous smooth cutting edge is formed at a periphery of an opening of the outer planing window. One end of the inner cutter tube is formed with an inner planing window. Two axially opposite sides of the inner planing window are each formed with one tooth portion, the two tooth portions being arranged opposite each other. A smooth cutting edge is formed at a periphery of an opening of the inner planing window.

A rotary drive handle according to the present invention is configured to be sleeved on a cutter tube assembly. The cutter tube assembly includes an inner cutter tube and an outer cutter tube sleeved on the inner cutter tube. The rotary drive handle comprises:
a drive assembly configured to drive the outer cutter tube to rotate relative to the inner cutter tube, the drive assembly including a drive sleeve and a handwheel, the drive sleeve being sleeved on the outer cutter tube and configured to rotate the outer cutter tube, the handwheel being sleeved on the drive sleeve and configured to rotate the drive sleeve;
a limiting assembly configured to restrict rotation of the drive sleeve and provide a detent feel for rotation of the outer cutter tube, the limiting assembly being fixedly sleeved on the cutter tube assembly, one end of the limiting assembly being inserted into the handwheel and engaged with the drive sleeve;
an elastic member configured to drive the handwheel to rotate and return, mounted between the drive sleeve and the handwheel.

Preferably, the limiting assembly includes an adapter sleeve. A plurality of limiting grooves are evenly distributed along a circumference of an outer wall of the adapter sleeve. The drive sleeve is formed with an elastic limiting cylinder matching the limiting grooves. A limiting protrusion is disposed inside the handwheel. When the drive sleeve is in a locked state, the limiting cylinder is engaged in one of the limiting grooves, and the limiting protrusion contacts and abuts the limiting cylinder.

An elastic member mounting groove and a protrusion sliding groove are formed on an outer wall of the drive sleeve. The elastic member is disposed in the elastic member mounting groove, and the limiting protrusion is slidably disposed in the protrusion sliding groove.

A front limiting sleeve is mounted between the drive sleeve and the cutter tube assembly, the front limiting sleeve being sleeved on the cutter tube assembly. A bushing is disposed inside the drive sleeve. A rear limiting sleeve is mounted between the limiting assembly and the cutter tube assembly, the rear limiting sleeve being sleeved on the cutter tube assembly. A fixed sleeve is mounted between the rear limiting sleeve and the front limiting sleeve, the fixed sleeve being sleeved on the cutter tube assembly.

A proximal end of the outer cutter tube is disposed inside the limiting assembly. The inner cutter tube extends through the limiting assembly and is connected to an external power mechanism.

A cutter head steering structure according to the present invention is configured to be sleeved on a cutter tube assembly. The cutter tube assembly includes an inner cutter tube and an outer cutter tube sleeved on the inner cutter tube. The cutter head steering structure comprises a handwheel, a fixed sleeve, a drive sleeve assembly, and a steel ball.

The drive sleeve assembly is fixedly sleeved on the outer cutter tube and configured to drive the outer cutter tube to rotate relative to the inner cutter tube.

The handwheel is drivingly connected to the drive sleeve assembly. Rotation of the handwheel drives the drive sleeve assembly to rotate. The handwheel is sleeved on the fixed sleeve.

The steel ball is disposed inside the handwheel and configured to restrict rotation of the drive sleeve assembly.

The fixed sleeve is fixedly disposed and configured to maintain a position of the steel ball and restrict axial sliding of the drive sleeve assembly. A spring is disposed between the handwheel and the fixed sleeve and configured to drive the handwheel to slide and return.

Preferably, the drive sleeve assembly includes a directional sleeve and a retaining ring. The retaining ring is fixedly sleeved on the directional sleeve. Rotation of the retaining ring drives the directional sleeve to rotate. The directional sleeve is sleeved on the outer cutter tube. Rotation of the directional sleeve drives the outer cutter tube to rotate.

A plurality of arc-shaped grooves are evenly distributed along a circumference of an outer wall of one end of the directional sleeve. A tapered cavity is defined inside the handwheel. When the outer cutter tube is in a locked state, one end of the steel ball is disposed in one of the arc-shaped grooves, and an inner wall of the tapered cavity contacts and abuts the steel ball.

A mounting ring is fixedly sleeved on an outer wall of one end of the directional sleeve. The plurality of arc-shaped grooves are formed on the mounting ring. An end of the fixed sleeve proximate to the directional sleeve is formed with a snap ring. The mounting ring is disposed inside the fixed sleeve, and the snap ring contacts and abuts one end of the mounting ring.

A clamping groove is formed on an outer side wall of the retaining ring. A through hole is formed in the handwheel at a position corresponding to the clamping groove. A pin shaft is inserted into the through hole and slidably disposed in the clamping groove.

A steel ball hole is formed in the fixed sleeve. The steel ball is inserted into the steel ball hole.

An adapter sleeve is sleeved on the cutter tube assembly. A proximal end of the outer cutter tube is disposed in the adapter sleeve. A proximal end of the inner cutter tube extends outwardly from the adapter sleeve. The fixed sleeve is fixedly sleeved on the adapter sleeve. One end surface of the adapter sleeve contacts and abuts the drive sleeve assembly.

A power handle according to the present invention is configured to be sleeved on a cutter tube assembly. The cutter tube assembly includes an inner cutter tube and an outer cutter tube sleeved on the inner cutter tube. The power handle comprises:
a drive assembly configured to drive the outer cutter tube to rotate relative to the inner cutter tube, the drive assembly including a handwheel, a retaining sleeve, and a steering sleeve, the steering sleeve being sleeved on the outer cutter tube and configured to rotate the outer cutter tube, the retaining sleeve being sleeved on the steering sleeve and configured to rotate the steering sleeve, the handwheel being sleeved on the retaining sleeve and configured to rotate the retaining sleeve;
a steel ball configured to restrict rotation of the steering sleeve;
a torsion spring configured to drive the handwheel to rotate and return, disposed between the retaining sleeve and the handwheel;
a fixed sleeve configured to maintain a position of the steel ball and restrict axial sliding of the steering sleeve, fixedly mounted inside the handwheel.

Preferably, a plurality of steel ball grooves are evenly distributed along a circumference of an outer wall of the steering sleeve. A steel ball relief groove is formed on an inner wall of the handwheel. Both the steel ball grooves and the steel ball relief groove are configured to receive the steel ball.

A ball-pressing protrusion is disposed on an inner wall of the handwheel and configured to press the steel ball tightly. The ball-pressing protrusion is disposed adjacent to the steel ball relief groove.

The retaining sleeve includes a baffle and a connecting tube that are coaxially fixed to each other. A limiting notch is formed in the baffle. A limiting protrusion is disposed on an inner wall of the handwheel and slidably disposed in the limiting notch. The connecting tube is disposed inside the handwheel. The torsion spring is sleeved on the connecting tube.

A limiting groove is formed in the connecting tube. A limiting post is disposed on the steering sleeve at a position corresponding to the limiting groove and inserted into the limiting groove.

A slotted ring is disposed on the steering sleeve. The steel ball groove is formed in the slotted ring. The slotted ring is disposed inside the fixed sleeve. A limiting ring is disposed inside one end of the fixed sleeve. One end of the limiting ring contacts and abuts one end of the slotted ring.

A steel ball hole is formed in the fixed sleeve. The steel ball is inserted into the steel ball hole.

The power handle further comprises a speed reducer. A proximal end of the inner cutter tube is connected to the speed reducer. A proximal end of the outer cutter tube is disposed outside the speed reducer. A hollow interior of the inner cutter tube forms a liquid suction passageway. A gap between the inner cutter tube and the outer cutter tube forms a water injection passageway.

Compared with the prior art, the present invention provides the following advantageous effects:
1. The present application integrates the cutter tube assembly, power assembly, negative pressure suction assembly, and water injection assembly into a single housing to form an integrated structure that eliminates an additional handle, facilitating cleaning and maintenance. The power assembly drives the cutter tube assembly to rotate for planing at a target site of a patient. The negative pressure suction assembly aspirates fragmented tissue generated during planing. The water injection assembly infuses fluid to the target site. Multiple functions are integrated into one integrated planing cutter, adapting to diverse working scenarios and offering high applicability.
2. The present application increases the contact area between the motor and the lateral mounting portion of the mounting base by forming the contact surface between the lateral mounting portion of the mounting base and the motor as a concave arcuate surface, thereby enhancing cooling performance for the motor.
3. The driving gear and dual transmission gear of the present application each adopt a single-side support structure. Compared with a double-side support structure, the single-side support structure simplifies assembly by eliminating coaxial positioning requirements.
4. The present application disposes seals at the connection between the inner cutter tube and the lateral mounting portion of the mounting base and at the rotational connection between the regulating valve and the lateral mounting portion of the mounting base, preventing leakage of aspirated fluid during negative pressure suction and improving suction efficiency of the negative pressure suction passageway.
5. The present application achieves positioning and mounting of the limiting tube by causing one end of the limiting tube to abut the rear end surface of the lateral mounting portion of the mounting base and the other end to abut a reinforcing rib inside the housing, thereby improving structural stability of the negative pressure suction passageway.
6. The present application achieves a sharper cutting edge and cleaner cutting by inclining the cutting edge of the inner cutter head inwardly and the cutting edge of the outer cutter head outwardly, and reducing the circumferential surface of the tooth portions of the outer cutter head.
7. The present application provides a shearing grip via a single-tooth structure on two opposite sides of the inner planing window. The cutting edges of both the inner planing window and the outer planing window are continuous with minimal burrs, sharp, and easy to manufacture.
8. The present application uses the outer cutter tube to semi-enclose the drill bit as a protective sheath, isolating the drill bit from healthy tissue and reducing trauma.
9. The present application achieves axial limitation of the rotating drill bit by causing the power adapter sleeve to abut and limit one end of the drill bit connection cylinder, and forming the other end of the rotating drill bit with an abutment surface that abuts and limits the other end of the drill bit connection cylinder.
10. The planing cutter of the present application enables relative rotation of the outer cutter tube with respect to the inner cutter tube via rotation of the handwheel. The water injection passageway flushes and cools the planing site, and the suction passageway aspirates waste fluid from the planing site, significantly improving planing efficiency.

### BRIEF DESCRIPTION OF THE DRAWINGS

Other features, objects, and advantages of the present invention will become more apparent from the detailed description of non-limiting embodiments with reference to the accompanying drawings.
FIG. 1 is a cross-sectional view schematically illustrating an overall structure of an integrated planing cutter according to Embodiment 1 of the present invention;
FIG. 2 is a cross-sectional view schematically illustrating an internal structure of a housing of the integrated planing cutter according to Embodiment 1 of the present invention;
FIG. 3 is a schematic view schematically illustrating an overall structure of a power assembly according to Embodiment 1 of the present invention;
FIG. 4 is a schematic view schematically illustrating a water injection assembly of the integrated planing cutter according to Embodiment 1 of the present invention;
FIG. 5 is a schematic view schematically illustrating one cutter head structure of the planing cutter according to Embodiment 1 of the present invention;
FIG. 6 is an exploded schematic view schematically illustrating another cutter head structure of the planing cutter according to Embodiment 1 of the present invention;
FIG. 7 is a schematic view schematically illustrating yet another cutter head structure of the planing cutter according to Embodiment 1 of the present invention;
FIG. 8 is a schematic view schematically illustrating an overall structure of a drill bit according to Embodiment 1 of the present invention;
FIG. 9 is a cross-sectional view schematically illustrating an overall structure of a planing cutter head according to Embodiment 2 of the present invention;
FIG. 10 is a cross-sectional view schematically illustrating a mounting structure of a rotating cutter head according to Embodiment 2 of the present invention;
FIG. 11 is a schematic view schematically illustrating structures of the rotating cutter head and a cutter head connection cylinder according to Embodiment 2 of the present invention;
FIG. 12 is an exploded structural schematic view schematically illustrating a rotary drive handle according to Embodiment 3 of the present invention;
FIG. 13 is a cross-sectional structural schematic view schematically illustrating the rotary drive handle according to Embodiment 3 of the present invention;
FIG. 14 is a structural schematic view schematically illustrating the rotary drive handle according to Embodiment 3 of the present invention;
FIG. 15 is an enlarged structural schematic view schematically illustrating a handwheel according to Embodiment 3 of the present invention;
FIG. 16 is an enlarged structural schematic view schematically illustrating a drive sleeve according to Embodiment 3 of the present invention;
FIG. 17 is an enlarged structural schematic view schematically illustrating an adapter sleeve according to Embodiment 3 of the present invention;
FIG. 18 is a cross-sectional structural schematic view schematically illustrating a cutter head steering structure according to Embodiment 4 of the present invention;
FIG. 19 is an enlarged structural schematic view schematically illustrating a portion A in FIG. 18 according to Embodiment 4 of the present invention;
FIG. 20 is a structural schematic view schematically illustrating the cutter head steering structure according to Embodiment 4 of the present invention;
FIG. 21 is an exploded structural schematic view schematically illustrating the cutter head steering structure according to Embodiment 4 of the present invention;
FIG. 22 is a cross-sectional structural schematic view schematically illustrating a power handle according to Embodiment 5 of the present invention;
FIG. 23 is an enlarged structural schematic view schematically illustrating a portion A in FIG. 22 according to Embodiment 5 of the present invention;
FIG. 24 is an exploded structural schematic view schematically illustrating the power handle according to Embodiment 5 of the present invention;
FIG. 25 is an enlarged structural schematic view schematically illustrating a retaining sleeve according to Embodiment 5 of the present invention;
FIG. 26 is an enlarged structural schematic view schematically illustrating a handwheel according to Embodiment 5 of the present invention;
FIG. 27 is an enlarged structural schematic view schematically illustrating a steering sleeve according to Embodiment 5 of the present invention;
FIG. 28 is an enlarged structural schematic view schematically illustrating a fixed sleeve according to Embodiment 5 of the present invention;
FIG. 29 is a structural schematic view schematically illustrating a planing cutter according to Embodiment 5 of the present invention;
FIG. 30 is a cross-sectional structural schematic view schematically illustrating the planing cutter according to Embodiment 5 of the present invention.

### Reference Numerals in Drawings

110 Inner cutter tube; 120 Outer cutter tube; 1001 Housing; 1002 Mounting base; 1003 Motor; 1004 Driving gear; 1005 Dual transmission gear; 1006 Driven gear; 1007 Fixed connection sleeve; 1008 Bearing; 1009 Seal; 1010 Negative pressure suction tube; 1011 Regulating valve; 1012 Limiting tube; 1013 Water injection tube; 1014 Water injection connection hole; 1015 Outer planing window; 1016 Inner planing window; 1017 Drill bit; 1018 Radial suction bore; 1019 Soft spring section; 2001 Rotating cutter head; 2002 Cutter head connection cylinder; 2003 Power adapter sleeve; 2004 Abutment surface; 2005 Water outlet hole; 2006 Suction bore; 3210 Drive sleeve; 3211 Limiting cylinder; 3212 Elastic member mounting groove; 3213 Protrusion sliding groove; 3214 Locking block; 3220 Handwheel; 3221 Limiting protrusion; 3310 Adapter sleeve; 3311 Limiting groove; 3400 Elastic member; 3510 Front limiting sleeve; 3520 Bushing; 3530 Rear limiting sleeve; 3540 Fixed sleeve; 3550 Seal; 4010 Handwheel; 4011 Tapered cavity; 4012 Through hole; 4020 Fixed sleeve; 4021 Snap ring; 4022 Steel ball hole; 4030 Steel ball; 4040 Spring; 4051 Directional sleeve; 4511 Mounting ring; 4512 Arc-shaped groove; 4052 Retaining ring; 40521 Clamping groove; 4060 Pin shaft; 4070 Adapter sleeve; 5210 Handwheel; 5211 Steel ball relief groove; 5212 Ball-pressing protrusion; 5213 Limiting protrusion; 5220 Retaining sleeve; 5221 Baffle; 52211 Limiting notch; 5222 Connecting tube; 5223 Limiting groove; 5230 Steering sleeve; 5231 Steel ball groove; 5232 Slotted ring; 5520 Limiting ring; 5300 Steel ball; 5400 Torsion spring; 5500 Fixed sleeve; 5510 Steel ball hole; 5600 Speed reducer; 5610 Adapter sleeve; 5620 Seal; 5710 Front housing; 5720 Rear housing; 5800 Suction passageway; 5810 Suction connector; 5820 Suction tube; 5900 Water injection passageway; 5910 Water injection connector; 5920 Water injection tube; 5930 Right-angle connector.

### DETAILED DESCRIPTIONS OF THE EMBODIMENTS

The present invention is described in detail below with reference to preferred embodiments. These embodiments enable those skilled in the art to better understand the present invention but do not limit the scope of the invention in any form. It should be noted that those skilled in the art can make various modifications and improvements without departing from the spirit of the invention. Such modifications and improvements are considered within the scope of protection defined by the appended claims.

### Embodiment 1

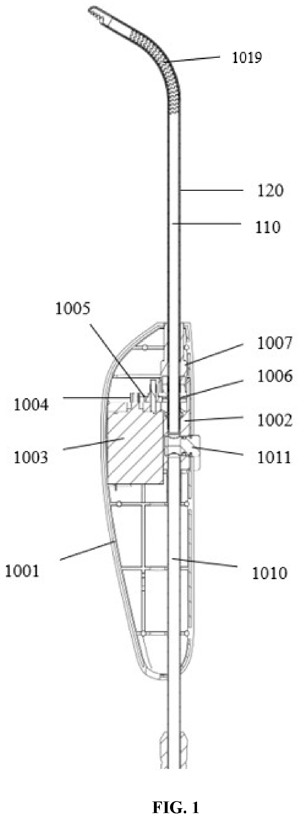

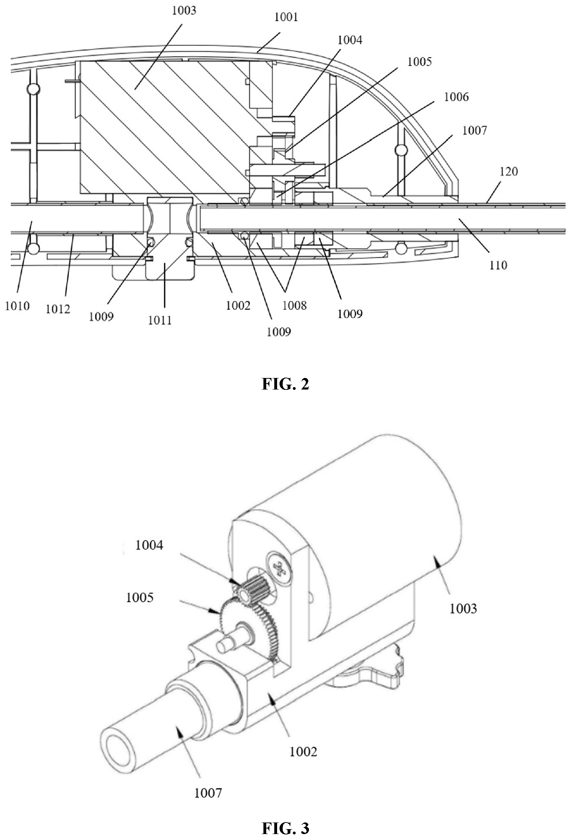

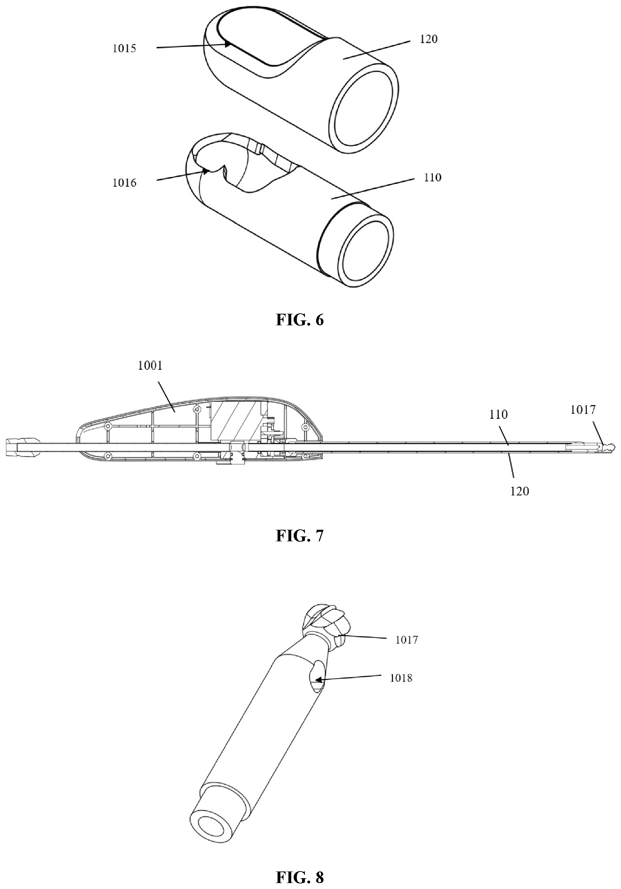

A planing cutter in accordance with the present invention, as illustrated in Figures 1 through 8, comprises a cutter tube assembly including an inner cutter tube 110 and an outer cutter tube 120, wherein the inner cutter tube 110 is rotatable relative to the outer cutter tube 120. Specifically, the planing cutter further comprises a housing 1001, a power assembly, a negative pressure suction assembly, and a water injection assembly. The power assembly is disposed within the housing 1001. One end of the cutter tube assembly extends into the housing 1001, the outer cutter tube 120 is fixedly secured to the housing 1001, and the inner cutter tube 110 is drivingly connected to an output end of the power assembly. One end of the negative pressure suction assembly extends into the housing 1001 and communicates with a cavity of the inner cutter tube 110, and an opposite end of the negative pressure suction assembly extends outwardly from the housing 1001. An end of the cutter tube assembly distal from the housing 1001 defines a water outlet. A water injection gap is defined between an inner wall of the outer cutter tube 120 and an outer wall of the inner cutter tube 110, the water injection gap being in communication with the water outlet. One end of the water injection assembly extends into the housing 1001 and communicates with the water injection gap between the outer cutter tube 120 and the inner cutter tube 110, and an opposite end of the water injection assembly extends outwardly from the housing 1001.

The planing cutter of the present application integrates the cutter tube assembly, power assembly, negative pressure suction assembly, and water injection assembly into the housing 1001 to form a unitary structure that dispenses with an additional handle, thereby facilitating cleaning and maintenance. Furthermore, the power assembly drivingly rotates the cutter tube assembly to perform a planing procedure at a target site of a patient. The negative pressure suction assembly aspirates fragmented tissue generated during planing, and the water injection assembly infuses fluid to the target site of the patient. Multiple functions are thus integrated into a unitary planing cutter, enabling adaptation to a wider range of working scenarios and providing high applicability.

Specifically, the housing 1001 of the present application is formed of two opposing half-shells, and opposing sides of the two half-shells are recessed to cooperatively define a receiving cavity. Further, reinforcing ribs are disposed within cavities of the two half-shells to retain devices integrated in the housing 1001, and the reinforcing ribs also increase structural strength of the housing 1001.

More specifically, the power assembly includes a mounting base 1002, a motor 1003, and a gear transmission structure. The mounting base 1002 is fixedly mounted within the housing 1001. It will be appreciated that the mounting base 1002 may be retained and secured within the housing 1001 either by the reinforcing ribs or by fasteners. The present application preferably retains and mounts the mounting base 1002 via the reinforcing ribs.

The mounting base 1002 includes a lateral mounting portion and a vertical mounting portion. The lateral mounting portion of the mounting base 1002 principally supports the motor 1003, while the vertical mounting portion of the mounting base 1002 principally provides a mounting location and support for the gear transmission structure, such that the overall structure is simple and easy to manufacture. The mounting base 1002 defines an axially extending through-hole that permits suction by the negative pressure suction assembly. During suction by the negative pressure suction assembly, fragmented tissue is sequentially withdrawn through the cavity of the inner cutter tube 110, the through-hole within the mounting base 1002, and the negative pressure suction tube 1010. This configuration achieves aspiration of fragmented tissue and, simultaneously, cools the mounting base 1002 and thereby further cools the motor 1003.

Specifically, the motor 1003 is seated on the lateral mounting portion of the mounting base 1002, an output end of the motor 1003 abuts the vertical mounting portion of the mounting base 1002, and an output shaft of the motor 1003 extends through the vertical mounting portion of the mounting base 1002. In one embodiment, the output end of the motor 1003 is fastened to the vertical mounting portion of the mounting base 1002 via fasteners. Further, a contact surface between the lateral mounting portion of the mounting base 1002 and the motor 1003 is a concave arcuate surface, which increases a contact area between the motor 1003 and the lateral mounting portion of the mounting base 1002, thereby enhancing cooling performance for the motor 1003.

The gear transmission structure includes a driving gear 1004, a dual transmission gear 1005, and a driven gear 1006. The driving gear 1004 is coaxially fixed to the output shaft of the motor 1003. The dual transmission gear 1005 is rotatably mounted to the vertical mounting portion of the mounting base 1002 via a pivot shaft. The driven gear 1006 is drivingly connected to the inner cutter tube 110. The dual transmission gear 1005 drivingly interconnects the driving gear 1004 and the driven gear 1006. It will be noted that one end of the pivot shaft of the dual transmission gear 1005 is connected to the vertical mounting portion of the mounting base 1002, and an opposite end of the pivot shaft of the dual transmission gear 1005 is fixedly connected to a retaining sleeve. The dual transmission gear 1005 is rotatably engaged with the pivot shaft. It will be further noted that both the driving gear 1004 and the dual transmission gear 1005 of the present application are single-side supported structures. Relative to a double-side supported structure, the single-side supported structure of the present application simplifies assembly by eliminating coaxial positioning requirements.

More specifically, the outer cutter tube 120 is fixedly connected to a front end of the lateral mounting portion of the mounting base 1002 via a fixed connection sleeve 1007. One end of the fixed connection sleeve 1007 extends into the front end of the lateral mounting portion of the mounting base 1002 and is fixedly secured thereto. An opposite end of the fixed connection sleeve 1007 extends to a front end of the housing 1001 and is fixedly secured to the housing 1001. An end of the outer cutter tube 120 extending into the housing 1001 extends into the fixed connection sleeve 1007 and is fixedly secured to the fixed connection sleeve 1007. The fixed connection sleeve 1007 defines an axial through-hole through which the inner cutter tube 110 passes, and the inner cutter tube 110 is rotatable relative to the fixed connection sleeve 1007. The mounting base 1002 defines a relief groove that permits meshing of the dual transmission gear 1005 with the driven gear 1006 and ensures that motion of the gear transmission structure does not interfere with the mounting base 1002.

The fixed connection sleeve 1007 is located forward of the relief groove, and a seal 1009 and a bearing 1008 are disposed between an end of the fixed connection sleeve 1007 proximate the mounting base 1002 and the inner cutter tube 110. Both the seal 1009 and the bearing 1008 are received in the axial through-hole of the fixed connection sleeve 1007. A bearing 1008 and a seal 1009 are disposed between a portion of the inner cutter tube 110 rearward of the relief groove and the axially extending through-hole within the mounting base 1002, thereby providing stable support for the inner cutter tube 110 and improving rotational stability of the inner cutter tube 110. Seals 1009 disposed forward and rearward of the relief groove prevent ingress of external fluid into the gear transmission structure, thereby helping to extend service life of the gear transmission structure.

The negative pressure suction assembly includes a negative pressure suction tube 1010 and a regulating valve 1011. The negative pressure suction tube 1010 is a straight tube. An end of the inner cutter tube 110 extending into the housing 1001 extends into the lateral mounting portion of the mounting base 1002, i.e., into the axial through-hole of the mounting base 1002. One end of the negative pressure suction tube 1010 extends into the lateral mounting portion of the mounting base 1002, i.e., into the axial through-hole of the mounting base 1002, and an opposite end of the negative pressure suction tube 1010 extends outwardly from the housing 1001, thereby establishing communication between the cavity of the inner cutter tube 110 and a cavity of the negative pressure suction tube 1010.

The regulating valve 1011 is rotatably mounted to the lateral mounting portion of the mounting base 1002 and is configured to connect or disconnect the negative pressure suction tube 1010 and the inner cutter tube 110. A regulating portion of the regulating valve 1011 is located externally of the housing 1001. Seals 1009 are disposed at a connection between the inner cutter tube 110 and the lateral mounting portion of the mounting base 1002 and at a rotational connection between the regulating valve 1011 and the lateral mounting portion of the mounting base 1002. The seals prevent leakage of aspirated fluid from the connection between the regulating valve 1011 and the mounting base 1002 and from the connection between the inner cutter tube 110 and the mounting base 1002 during negative pressure suction, thereby helping to improve suction efficiency of the negative pressure suction passage.

Since the negative pressure suction tube 1010 is connected to external equipment during use, a tensile force is exerted on the negative pressure suction tube 1010. To prevent failure of the connection between the negative pressure suction tube 1010 and the mounting base 1002, the present application provides a reliable mounting arrangement: a retaining tube 1012 is coaxially received over the negative pressure suction tube 1010. One end of the retaining tube 1012 abuts a rear end surface of the lateral mounting portion of the mounting base 1002, and an opposite end of the retaining tube 1012 abuts a reinforcing rib inside the housing 1001, thereby positioning and mounting the retaining tube 1012 and improving structural stability of the negative pressure suction passage.

The water injection assembly includes a water injection tube 1013. One end of the water injection tube 1013 is disposed inside the housing 1001, and an opposite end of the water injection tube 1013 extends outwardly from the housing 1001. The fixed connection sleeve 1007 defines a water injection port 1014. An end of the water injection tube 1013 inside the housing 1001 communicates with the water injection port 1014, and the water injection port 1014 communicates with the water injection gap between the outer cutter tube 120 and the inner cutter tube 110.

The end of the cutter tube assembly distal from the housing 1001 is provided with a water outlet in communication with the water injection gap. The water injection tube 1013 is connectable to an external water source. External water first flows through the water injection tube 1013, then into the water injection gap between the outer cutter tube 120 and the inner cutter tube 110, and thereafter exits through the water outlet at the distal end of the cutter tube assembly.

The present application discloses a planing cutter head: one end of the outer cutter tube 120 is formed with an outer planing window 1015, and the outer planing window 1015 is formed with outer planing teeth. The outer planing teeth include a plurality of continuous tooth portions, each tooth portion of the outer planing teeth being inclined outwardly. One end of the inner cutter tube 110 is formed with an inner planing window 1016, and the inner planing window 1016 is formed with inner planing teeth. The inner planing teeth include a plurality of continuous tooth portions, each tooth portion of the inner planing teeth being inclined inwardly. Further, the tooth portions of the outer planing teeth and the tooth portions of the inner planing teeth are staggered. Such a staggered arrangement enhances cutting performance of the cutter tube assembly. In the present embodiment, cutting edges of the inner cutter head are inclined inwardly, and cutting edges of the outer cutter head are inclined outwardly, with a circumferential surface of the outer cutter head tooth portions reduced, resulting in sharper cutting edges and more efficient cutting. The cutter tube assembly of the present embodiment is configured primarily for soft tissue. During negative pressure suction, fragmented tissue enters a lumen of the inner cutter tube 110 through the outer planing window 1015 and the inner planing window 1016 and is thereafter discharged through the cavity of the negative pressure suction tube 1010.

The present application discloses an alternative planing cutter head: one end of the outer cutter tube 120 is formed with an outer planing window 1015, and a periphery of an opening of the outer planing window 1015 forms a continuous smooth cutting edge. One end of the inner cutter tube 110 is formed with an inner planing window 1016, and two axially opposing sides of the inner planing window 1016 are each formed with one tooth portion, the two tooth portions being arranged in opposition. A periphery of an opening of the inner planing window 1016 forms a smooth continuous cutting edge. The single tooth portions on opposing sides of the inner planing window 1016 provide a gripping surface for cutting, and cutting edges of both the inner planing window 1016 and the outer planing window 1015 are continuous, substantially burr-free, sharp, and easy to manufacture. The cutter tube assembly of the present embodiment is configured primarily for soft tissue. During negative pressure suction, fragmented tissue enters the lumen of the inner cutter tube 110 through the outer planing window 1015 and the inner planing window 1016 and is thereafter discharged through the cavity of the negative pressure suction tube 1010.

The present application further discloses a drill bit structure that may replace the planing cutter heads of the foregoing embodiments. A drill bit 1017 is coaxially fixed to one end of the inner cutter tube 110. The drill bit 1017 is rotatable relative to the outer cutter tube 120, and an internal bore of the drill bit 1017 communicates with an internal passage of the inner cutter tube 110. The drill bit 1017 is formed with radial suction bores 1018 distributed circumferentially and evenly about the drill bit 1017. Each radial suction bore 1018 communicates with the internal bore of the drill bit 1017, and the outer cutter tube 120 partially surrounds the drill bit 1017. In a preferred embodiment, the drill bit 1017 is formed with two symmetric radial suction bores 1018, both in communication with the internal bore of the drill bit 1017, which helps improve rotational stability and dynamic balance of the drill bit 1017. The drill bit 1017 of the present embodiment is configured primarily for hard tissue such as bone. During negative pressure suction, fragmented tissue enters the internal bore of the drill bit 1017 through the radial suction bores 1018, then into the lumen of the inner cutter tube 110, and is thereafter discharged through the cavity of the negative pressure suction tube 1010. The outer cutter tube 120 partially surrounding the drill bit 1017 acts as a protective sheath, isolating the drill bit 1017 from healthy tissue and reducing trauma to healthy tissue. It will be noted that the drill bit 1017 of the present embodiment may omit a water injection function.

The unitary planing cutter of the present application planes tissue at a patient's target site, aspirates fragmented tissue generated during planing via negative pressure suction, and infuses fluid to the target site. With the regulating valve 1011, the negative pressure suction passage may be closed when operating in a confined space such as a joint, preventing accidental drainage of fluid from the confined space through the negative pressure suction passage, thereby achieving controllable suction and adapting to more working scenarios. Configuration of the inner cutter tube 110 and outer cutter tube 120 improves planing performance. The radial suction bores 1018 distributed circumferentially and evenly about the drill bit 1017 improve dynamic balance of the drill bit 1017.

It will be further noted that the cutter tube assembly of the present application may have a straight configuration, or may have a straight portion proximate the housing 1001 and a curved portion distal from the housing 1001. A straight configuration enables smoother negative pressure suction. Where the cutter tube assembly has a curved distal portion, the curved section of the inner cutter tube is formed as a flexible torque-transmitting pipeline structure, such as a flexible spring section 1019 similar to a flexible coil structure. Further, a protective sleeve may be received over the flexible spring section 1019.

It will be further noted that the unitary planing cutter of the present application may integrate cables and/or plugs, and the motor 1003 may be powered and controlled via the cables and plugs integrated on the housing of the unitary planing cutter. In one embodiment, the unitary planing cutter of the present application is connected to a main unit via cables and/or plugs, and planing operation or stoppage of the unitary planing cutter is controlled via a foot pedal.

### Embodiment 2

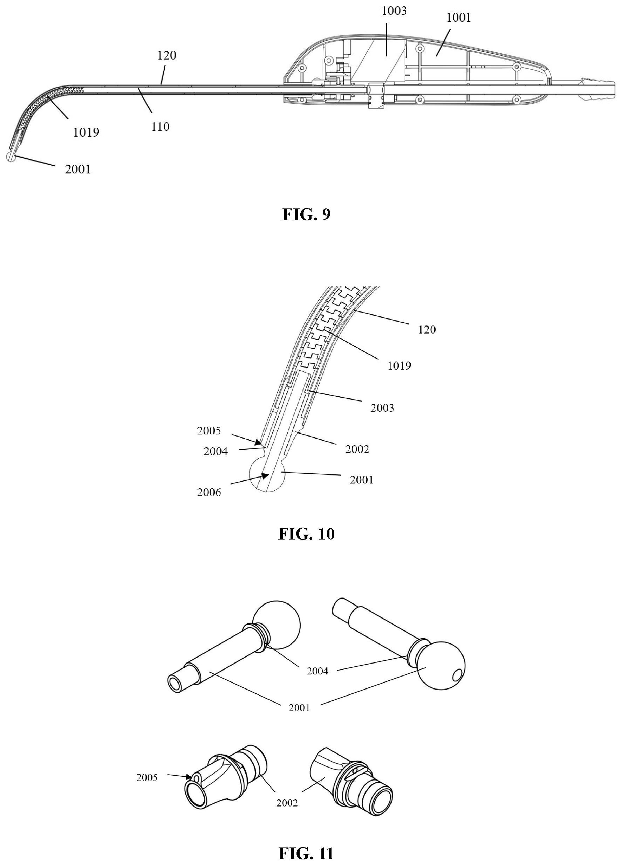

Based on Embodiment 1, a planing drill bit assembly in accordance with the present invention is improved primarily in the cutter tube assembly and the water injection gap of the cutter head assembly. As illustrated in Figures 9 through 11, the planing drill bit assembly comprises a cutter tube assembly including an inner cutter tube 110 and an outer cutter tube 120, wherein the inner cutter tube 110 is rotatable relative to the outer cutter tube 120. The planing drill bit assembly further comprises a rotating drill bit 2001, a drill bit connection cylinder 2002, and a power adapter sleeve 2003. One end of the drill bit connection cylinder 2002 extends into the outer cutter tube 120 and is fixedly secured to the outer cutter tube 120. One end of the rotating drill bit 2001 extends through an interior of the drill bit connection cylinder 2002 and into the inner cutter tube 110. The rotating drill bit 2001 is coaxially fixed to the inner cutter tube 110 via the power adapter sleeve 2003. The rotating drill bit 2001 is rotatably engaged with the drill bit connection cylinder 2002. The power adapter sleeve 2003 abuts and retains one end of the drill bit connection cylinder 2002, and an opposite end of the rotating drill bit 2001 is formed with an abutment surface 2004 that abuts and retains an opposite end of the drill bit connection cylinder 2002.

In the present embodiment, the rotating drill bit 2001 acts on hard tissue. The present application supports the rotating drill bit 2001 using the drill bit connection cylinder 2002 fixedly connected to the outer cutter tube. The rotating drill bit 2001 is drivingly connected to the inner cutter tube 110 via the power adapter sleeve 2003. The power adapter sleeve 2003 abuts and retains one end of the drill bit connection cylinder 2002, and the rotating drill bit 2001 is formed with the abutment surface 2004 that abuts and retains an opposite end of the drill bit connection cylinder 2002, thereby axially retaining the rotating drill bit 2001. In particular, where the cutter tube assembly has a curved distal portion, the curved section of the inner cutter tube is configured to transmit torque and is formed as a soft spring section. Since the soft spring section is prone to axial elongation, absent axial retention of the rotating drill bit 2001, the rotating drill bit 2001 would shift axially during a surgical procedure, adversely affecting surgical results.

It will be noted that the outer cutter tube 120 includes a double-layer wall, and a water injection gap is defined between the double-layer wall of the outer cutter tube 120. The drill bit connection cylinder 2002 is formed with a water outlet hole 2005 in communication with the water injection gap between the double-layer wall. The rotating drill bit 2001 is formed with a suction bore 2006 coaxial with the rotating drill bit 2001. The suction bore 2006 is a through-hole having an opening at a working end face of the rotating drill bit 2001. The suction bore 2006 communicates with an inner cavity of the inner cutter tube 110. Water is injected through the water injection gap defined by the double-layer wall of the outer cutter tube 120, thereby isolating the water injection passage from the soft spring section of the inner cutter tube 110. This prevents fluid in the water injection gap from being aspirated during negative pressure suction, improving reliability and stability of water injection.

It will be further noted that a portion of the drill bit connection cylinder 2002 extending outwardly from the outer cutter tube 120 is tapered from an end proximate the outer cutter tube 120 to an end distal therefrom, which reduces visual obstruction and improves surgical visibility.

### Embodiment 3

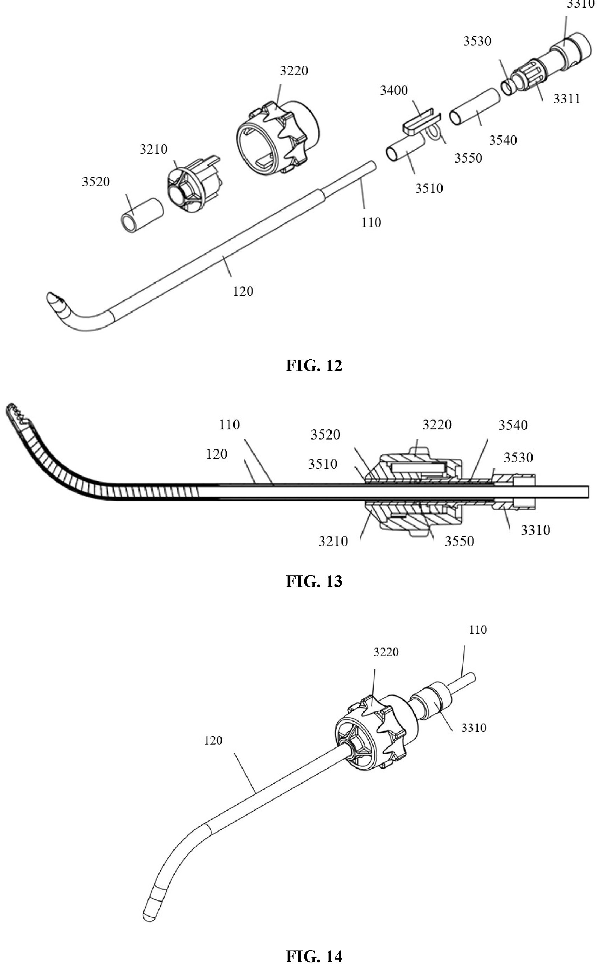

Referring to Figures 12 through 17, a rotary drive handle of the present embodiment is configured to be received over a cutter tube assembly. The cutter tube assembly of the present embodiment corresponds to the cutter tube assembly of Embodiment 1 or Embodiment 2 and includes an inner cutter tube 110 and an outer cutter tube 120 sleeved over the inner cutter tube 110. The rotary drive handle comprises a drive assembly, a retaining assembly, and an elastic member 3400, preferably a U-shaped elastic steel plate. The drive assembly is configured to drivingly rotate the outer cutter tube 120 relative to the inner cutter tube 110 and includes a drive sleeve 3210 and a handwheel 3220. The drive sleeve 3210 is sleeved over the outer cutter tube 120 such that rotation of the drive sleeve 3210 rotates the outer cutter tube 120. The handwheel 3220 is sleeved over the drive sleeve 3210 and is configured to drivingly rotate the drive sleeve 3210. The retaining assembly is configured to restrict rotation of the drive sleeve 3210 and provide a detent feel for rotation of the outer cutter tube 120. The retaining assembly is fixedly received over the cutter tube assembly, and one end of the retaining assembly is inserted into the handwheel 3220 and engaged with the drive sleeve 3210 to restrict rotation of the drive sleeve 3210. The elastic member 3400 is mounted between the drive sleeve 3210 and the handwheel 3220 to control rotational return of the handwheel 3220. A proximal end of the outer cutter tube 120 is disposed inside the retaining assembly, and the inner cutter tube 110 extends through the retaining assembly for connection to an external power mechanism.

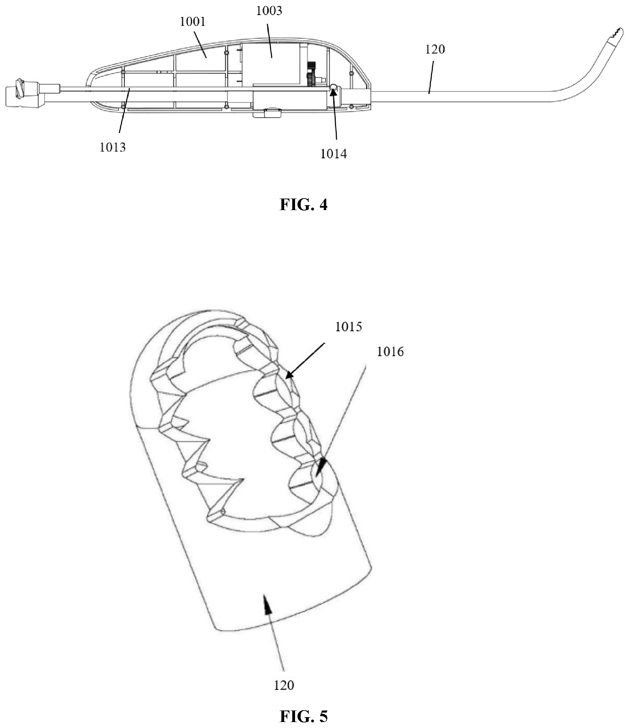

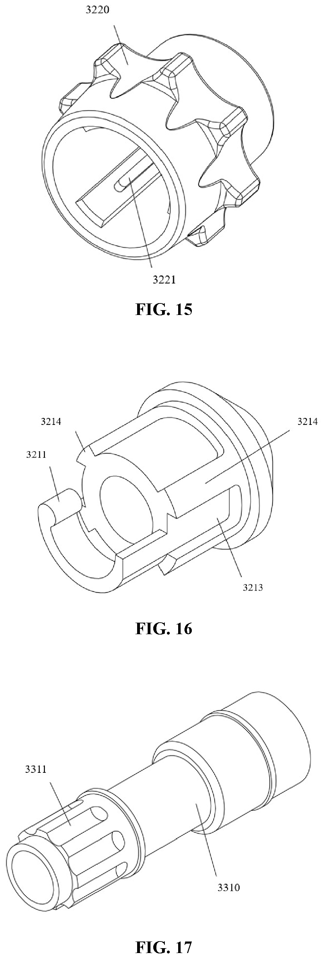

Specifically, referring to Figure 17, the retaining assembly includes an adapter sleeve 3310 having an outer wall which is located at one end of the adapter sleeve 310 and formed with a plurality of retaining grooves 3311 distributed circumferentially and evenly thereabout, the retaining grooves 3311 being arcuate. Referring to Figure 5, the drive sleeve 3210 is formed with a resilient retaining cylinder 3211 configured to mate with the retaining grooves 3311. Cooperation between the retaining cylinder 3211 and the retaining grooves 3311 engages the retaining assembly with the drive sleeve and simultaneously retains the retaining cylinder 3211 in a selected retaining groove 3311 to lock the drive sleeve against rotation, thereby locking the outer cutter tube. Referring to Figure 4, the handwheel 3220 has an inner wall formed with a retaining protrusion 3221. When the drive sleeve 3210 is in a locked state, the retaining cylinder 3211 is received in one of the retaining grooves 3311, and the retaining protrusion 3221 contacts and abuts the retaining cylinder 3211 to press the retaining cylinder 3211 firmly in the retaining groove 3311, thereby preventing rotation of the drive sleeve 3210 and thus rotation of the outer cutter tube 120.

Referring to Figure 5, an outer wall of the drive sleeve 3210 is formed with an elastic member mounting groove 3212 and a protrusion sliding groove 3213, preferably opposing each other. The elastic member 3400 is received in the elastic member mounting groove 3212, and the retaining protrusion 3220 is slidably received in the protrusion sliding groove 3213. Specifically, four locking blocks 3214 are arranged on the outer wall of the drive sleeve 3210 in two opposing pairs. Two of the locking blocks 3214 and the outer wall of the drive sleeve 3210 cooperatively define the elastic member mounting groove 3212, and the other two locking blocks 3214 and the outer wall of the drive sleeve 3210 cooperatively define the protrusion sliding groove 3213. Upon rotating the handwheel 3220 by a predetermined angle, the retaining protrusion 3221 disengages from the retaining cylinder 3211. Upon further rotation until the retaining protrusion 3221 inside the handwheel 3220 abuts a side wall of the protrusion sliding groove 3213, continued rotation of the handwheel 3220 drives the drive sleeve 3210 to rotate and thereby drives the outer cutter tube 120 to rotate relative to the inner cutter tube 110. Upon rotation to a desired angle, the retaining cylinder 3211 snaps into another retaining groove 3311. Releasing the handwheel 3220 allows the handwheel 3220 to return rotationally to an original position under action of the elastic member 3400. At this point, the retaining protrusion 3221 presses the retaining cylinder 3211 firmly into the other retaining groove 3311, thereby automatically locking the drive sleeve 3210 against unintended rotation.

To provide a more secure and stable connection between the drive sleeve 3210 and the cutter tube assembly, a front retaining sleeve 3510 is mounted between the drive sleeve 3210 and the cutter tube assembly and is received over the cutter tube assembly. A bushing 3520 is received over the front retaining sleeve 3510 and disposed inside the drive sleeve 3210. To provide a more secure and stable connection between the retaining assembly and the cutter tube assembly, a rear retaining sleeve 3530 is mounted between the retaining assembly and the cutter tube assembly and is received over the cutter tube assembly. A fixed sleeve 3540 is mounted between the rear retaining sleeve 3530 and the front retaining sleeve 3510 and is received over the cutter tube assembly. The fixed sleeve 3540 and the rear retaining sleeve 3530 further restrict axial sliding of the adapter sleeve 3310 along the cutter tube assembly. A seal 3550 is received over the fixed sleeve 3540 and disposed between the bushing 3520 and the adapter sleeve 3310.

When adjustment of a position of a planing window on the outer cutter tube 120 is needed, i.e., when rotation of the outer cutter tube 120 relative to the inner cutter tube 110 is needed, the handwheel 3220 is rotated. During rotation, the retaining protrusion 3221 gradually disengages from the retaining cylinder 3211. Upon rotation until the retaining protrusion 3221 inside the handwheel 3220 abuts the side wall of the protrusion sliding groove 3213, continued rotation of the handwheel 3220 drives the drive sleeve 3210 to rotate and thereby drives the outer cutter tube 120 to rotate relative to the inner cutter tube 110. Rotation of the drive sleeve 3210 withdraws the retaining cylinder 3211 from the retaining groove 3311. Upon rotation to a desired angle, the retaining cylinder 3211 snaps into another retaining groove 3311. Releasing the handwheel 3220 allows the handwheel 3220 to return rotationally to the original position under action of the elastic member 3400. At this point, the retaining protrusion 3221 presses the retaining cylinder 3211 firmly into the other retaining groove 3311, thereby automatically locking the drive sleeve 3210 against unintended rotation. The rotary drive handle of the present embodiment enables relative rotation of the outer cutter tube 120 with respect to the inner cutter tube 110 simply by rotating the handwheel 3220. The plurality of retaining grooves 3311 on the adapter sleeve 3310 enable a plurality of rotational detent positions, providing convenient and rapid operation.

Referring to Figures 12 through 17, a planing cutter of the present embodiment includes the rotary drive handle and the cutter tube assembly of Embodiment 1. Connecting a proximal end of the inner cutter tube 110 of the cutter tube assembly to a power mechanism enables rotational control of the inner cutter tube 110. Rotational control of the outer cutter tube 120 relative to the inner cutter tube 110 is enabled via the rotary drive handle. The planing cutter is convenient and flexible to use.

It will be noted that in the foregoing embodiments, the distal end refers to an end of the cutter tube assembly configured for planing bone tissue, and the proximal end refers to an end opposite the distal end, i.e., a handheld end.

### Embodiment 4

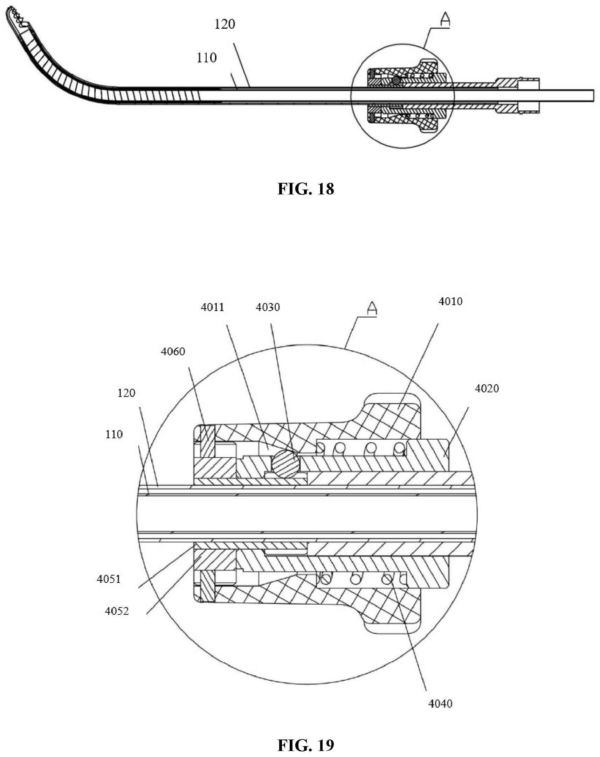

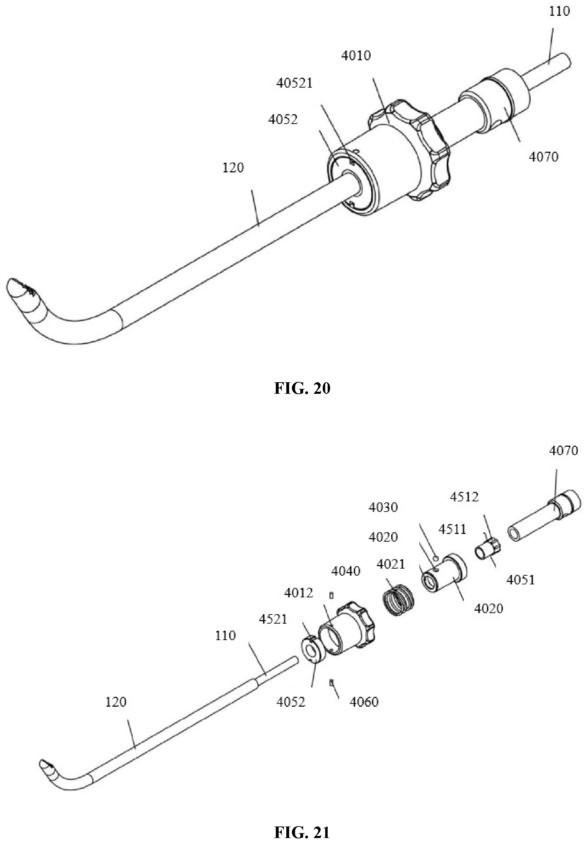

Referring to Figures 18 through 21, the present embodiment discloses a cutter head steering structure configured to be received over a cutter tube assembly. The cutter tube assembly of the present embodiment corresponds to the cutter tube assembly of Embodiment 1 or Embodiment 2 and includes an inner cutter tube 110 and an outer cutter tube 120 received over the inner cutter tube 110. The cutter head steering structure is configured to drivingly rotate the outer cutter tube 120 relative to the inner cutter tube 110.

The cutter head steering structure includes a handwheel 4010, a fixed sleeve 4020, a drive sleeve assembly, and a steel ball 4030. The drive sleeve assembly is fixedly received over the outer cutter tube 120 and is configured to drivingly rotate the outer cutter tube 120 relative to the inner cutter tube 110, such that rotation of the drive sleeve assembly rotates the outer cutter tube 120. The handwheel 4010 is drivingly connected to the drive sleeve assembly, such that rotation of the handwheel 4010 rotates the drive sleeve assembly. The handwheel 4010 is received over the fixed sleeve 4020. To facilitate rotation of the handwheel 4010, an outer circumference of the handwheel 4010 is formed with anti-slip grooves. The steel ball 4030 is disposed inside the handwheel 4010 and is configured to restrict rotation of the drive sleeve assembly. The fixed sleeve 4020 is fixedly mounted and is configured to maintain a position of the steel ball 4030 and restrict axial sliding of the drive sleeve assembly. A spring 4040 is disposed between the handwheel 4010 and the fixed sleeve 4020 and is configured to drivingly return the handwheel 4010 to an original position.

Specifically, the drive sleeve assembly includes a directional sleeve 4051 and a retaining ring 4052. The retaining ring 4052 is fixedly received over the directional sleeve 4051, such that rotation of the retaining ring 4052 rotates the directional sleeve 4051. The directional sleeve 4051 is received over the outer cutter tube 120, such that rotation of the directional sleeve 4051 rotates the outer cutter tube 120, whereby rotation of the drive sleeve assembly rotates the outer cutter tube 120. A plurality of arcuate grooves 4512 are distributed circumferentially and evenly about an outer wall of one end of the directional sleeve 4051. Specifically, the fixed sleeve 4020 is formed with a mounting ring 4511 on the outer wall of the end of the directional sleeve 4051, and the plurality of arcuate grooves 4512 are distributed evenly about the mounting ring 4511. The arcuate grooves 4512 are configured to receive the steel ball 4030. The handwheel 4010 defines a tapered cavity 4011 therein. When the outer cutter tube 120 is in a locked state, one end of the steel ball 4030 is received in one of the arcuate grooves 4512, and an inner wall of the tapered cavity 4011 contacts and abuts the steel ball 4030. An outer side wall of the retaining ring 4052 is formed with a locking groove 40521. The handwheel 4010 is formed with a through-hole 4012 at a location corresponding to the locking groove 40521. A pivot pin 4060 is inserted into the through-hole 4012 and slidably received in the locking groove 40521. An end of the retaining ring 4052 distal from the cutter head abuts an end of the fixed sleeve 4020 proximate the cutter head, further restricting axial sliding of the drive sleeve assembly. Sliding and rotating the handwheel 4010 away from the cutter head causes the handwheel 4010 to slide away from the cutter head, the pivot pin 4060 to slide within the locking groove 40521, and a wall of the tapered cavity 4011 inside the handwheel 4010 to disengage from the steel ball 4030, thereby relieving pressure on the steel ball 4030 and permitting rotation of the directional sleeve 4051. Since one end of the pivot pin 4060 is received in the locking groove 40521, rotation of the handwheel 4010 drives the retaining ring 4052 to rotate, which rotates the directional sleeve 4051 and thereby rotates the outer cutter tube 120. When the outer cutter tube 120 rotates to a desired position, the steel ball 4030 enters another arcuate groove 4512. Releasing the handwheel 4010 allows the handwheel 4010 to slide back toward the cutter head under restoring force of the spring 4040. The wall of the tapered cavity 4011 inside the handwheel 4010 again presses the steel ball 4030 firmly, thereby locking the directional sleeve 4051 and thus locking the outer cutter tube 120.

To prevent axial sliding of the directional sleeve 4051 of the drive sleeve assembly, an end of the fixed sleeve 4020 proximate the directional sleeve 4051 is formed with a snap ring 4021. The mounting ring 4511 is disposed inside the fixed sleeve 4020, and the snap ring 4021 contacts and abuts one end of the mounting ring 4511. An adapter sleeve 4070 is received over the cutter tube assembly for connection to an external gearbox of conventional design. A proximal end of the outer cutter tube 120 is disposed in the adapter sleeve 4070, and a proximal end of the inner cutter tube 110 extends outwardly from the adapter sleeve 4070. The fixed sleeve 4020 is fixedly mounted to the adapter sleeve 4070, and one end surface of the adapter sleeve 4070 contacts and abuts the drive sleeve assembly. The snap ring 4021 and the adapter sleeve 4070 cooperate to fix an axial position of the directional sleeve 4051 and prevent axial sliding during use. It will be noted that in the present embodiment, the distal end refers to a direction toward the cutter head of the cutter tube assembly, and the proximal end refers to a direction away from the cutter head.

The fixed sleeve 4020 is formed with a steel ball hole 4022, and the steel ball 4030 is received in the steel ball hole 4022. The steel ball hole 4022 stabilizes the steel ball 4030 and prevents sliding misalignment that could interfere with rotation of the outer cutter tube 120.

With the cutter head steering structure of the present embodiment, when rotation of the outer cutter tube 120 relative to the inner cutter tube 110 is needed, the handwheel 4010 is slid away from the cutter head until fully displaced. At this point, the pivot pin 4060 slides within the locking groove 40521, the spring 4040 is compressed, and the wall of the tapered cavity 4011 inside the handwheel 4010 disengages from the steel ball 4030, relieving pressure on the steel ball 4030 and permitting rotation of the directional sleeve 4051. Rotating the handwheel 4010 drives the retaining ring 4052 to rotate via the pivot pin 4060, which rotates the directional sleeve 4051 and thereby rotates the outer cutter tube 120 to achieve relative rotation of the outer cutter tube 120 with respect to the inner cutter tube 110. During rotation of the directional sleeve 4051, the steel ball 4030 withdraws from an original arcuate groove 4512. When the outer cutter tube 120 rotates to a desired position, the steel ball 4030 enters a corresponding arcuate groove 4512. Releasing the handwheel 4010 allows the handwheel 4010 to slide back toward the cutter head under restoring force of the spring 4040. The wall of the tapered cavity 4011 inside the handwheel 4010 again presses the steel ball 4030 firmly, thereby locking the directional sleeve 4051 and thus locking the outer cutter tube 120. Operation is simple and rapid, improving surgical efficiency and reducing surgical duration to a certain extent.

Referring to Figures 18 through 21, the present embodiment provides a planing cutter including the cutter head steering structure and the cutter tube assembly of Embodiment 1. Connecting a proximal end of the inner cutter tube 110 of the cutter tube assembly to an external power mechanism enables rotational control of the inner cutter tube 110. Rotational control of the outer cutter tube 120 relative to the inner cutter tube 110 is enabled by sliding and rotating the handwheel 4010. The planing cutter offers convenient and flexible operation.

### Embodiment 5

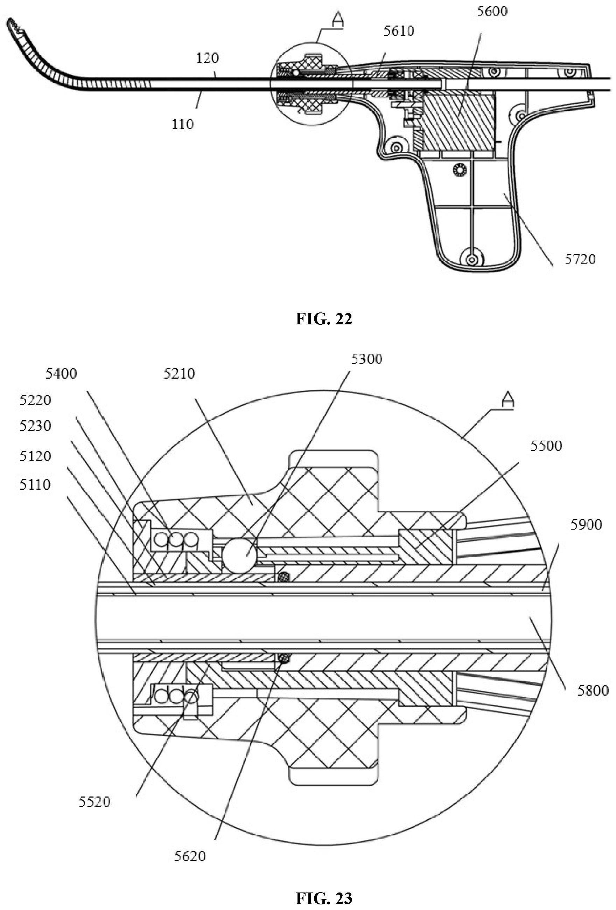

Referring to Figures 22 through 30, the present embodiment discloses a power handle configured to be received over a cutter tube assembly. The cutter tube assembly of the present embodiment corresponds to the cutter tube assembly of Embodiment 1 or Embodiment 2 and includes an inner cutter tube 110 and an outer cutter tube 120 received over the inner cutter tube 110. The power handle comprises a drive assembly, a steel ball 5300, a fixed sleeve 5500, and a torsion spring 5400. The drive assembly is configured to drivingly rotate the outer cutter tube 120 relative to the inner cutter tube 110 and includes a handwheel 5210, a retaining sleeve 5220, and a steering sleeve 5230. The steering sleeve 5230 is received over the outer cutter tube 120 and is configured to drivingly rotate the outer cutter tube 120. The retaining sleeve 5220 is received over the steering sleeve 5230 and is configured to drivingly rotate the steering sleeve 5230. The handwheel 5210 is received over the retaining sleeve 5220 and is configured to drivingly rotate the retaining sleeve 5220. The steel ball 5300 is configured to restrict rotation of the steering sleeve 5230. The torsion spring 5400 is configured to drivingly return the handwheel 5210 to an original rotational position and is disposed between the retaining sleeve 5220 and the handwheel 5210. The fixed sleeve 5500 is configured to maintain a position of the steel ball 5300 and restrict axial sliding of the steering sleeve 5230 and is fixedly mounted inside the handwheel 5210.

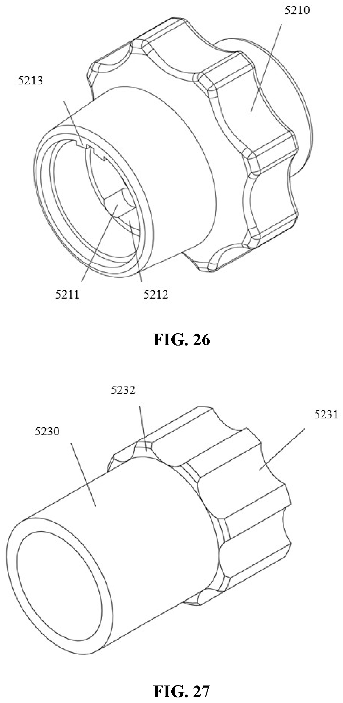

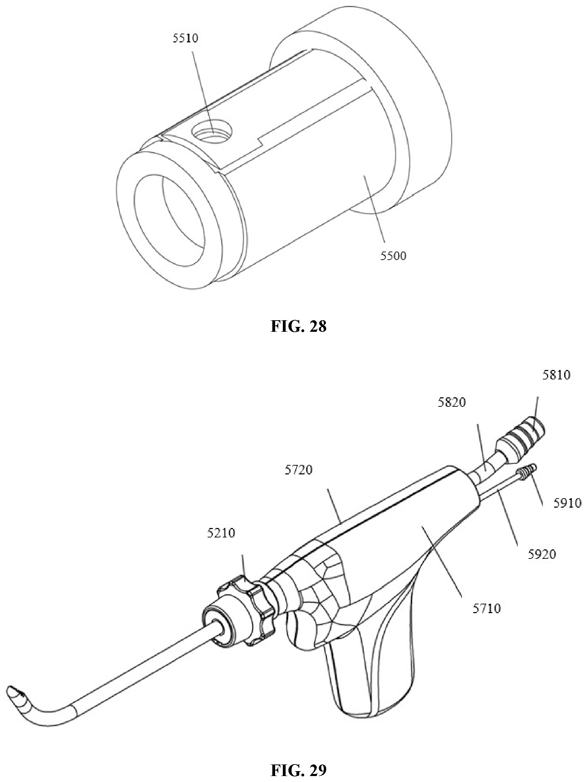

Specifically, referring to Figures 27 and 28, a plurality of steel ball grooves 5231 are distributed circumferentially and evenly about an outer wall of the steering sleeve 5230, and an inner wall of the handwheel 5210 is formed with a steel ball relief groove 5211. Both the steel ball grooves 5231 and the steel ball relief groove 5211 are configured to receive the steel ball 5300. The plurality of steel ball grooves 5231 enable a plurality of rotational detent positions for the outer cutter tube 120. The inner wall of the handwheel 5210 is formed with a ball-pressing protrusion 5212 configured to press the steel ball 5300 firmly. The ball-pressing protrusion 5212 is disposed adjacent the steel ball relief groove 5211. When the outer cutter tube 120 is in a locked non-rotating state, the ball-pressing protrusion 5212 presses the steel ball 5300 firmly into one of the steel ball grooves 5231.

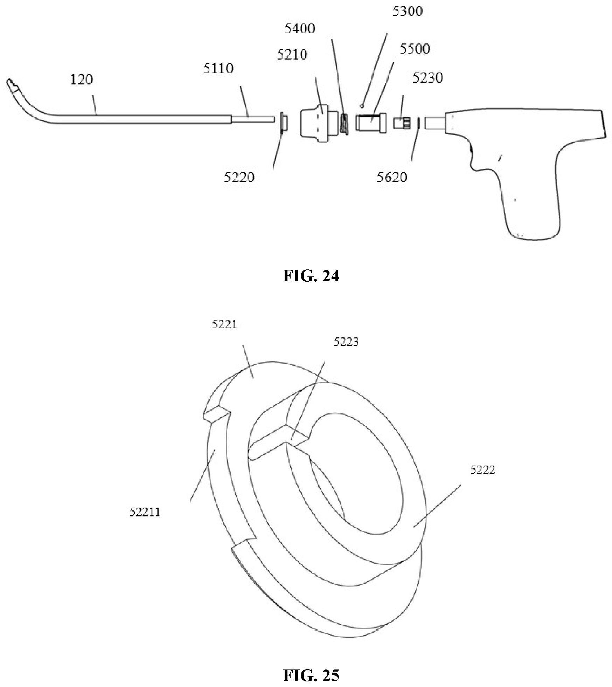

Referring to Figure 25, the retaining sleeve 5220 includes a baffle 5221 and a connecting tube 5222 that are coaxially fixed to each other. The baffle 5221 is formed with a retaining notch 52211, and the inner wall of the handwheel 5210 is formed with a retaining protrusion 5213 slidably received in the retaining notch 52211. With this configuration, upon rotating the handwheel 5210 to a predetermined angle where the retaining protrusion 5213 abuts an inner wall of the retaining notch 52211, the handwheel 5210 drives the retaining sleeve 5220 to rotate and thereby drives the steering sleeve 5230 to rotate. The connecting tube 5222 is disposed inside the handwheel 5210, and the torsion spring 5400 is received over the connecting tube 5222. The connecting tube 5222 is formed with a retaining groove 5223, and the steering sleeve 5230 is formed with a retaining post at a location corresponding to the retaining groove 5223, the retaining post being inserted into the retaining groove 5223. Insertion of the retaining post into the retaining groove 5223 couples the retaining sleeve 5220 to the steering sleeve 5230, such that rotation of the retaining sleeve 5220 rotates the steering sleeve 5230.

Referring to Figure 28, one end of the fixed sleeve 5500 is received over an end of the steering sleeve 5230 formed with the steel ball grooves 5231. Specifically, the steering sleeve 5230 is provided with a slotted ring 5232 in which the steel ball grooves 5231 are formed. The slotted ring 5232 is disposed inside the fixed sleeve 5500. An end of the fixed sleeve 5500 has an inner wall formed with a retaining ring 5520, and one end of the retaining ring 5520 contacts and abuts one end of the slotted ring 5232. Contact and abutment between the retaining ring 5520 inside the fixed sleeve 5500 and the slotted ring 5232 on the steering sleeve 5230 prevents axial sliding of the steering sleeve 5230, thereby stabilizing rotational motion of the outer cutter tube 120 relative to the inner cutter tube 110. The fixed sleeve 5500 is formed with a steel ball hole 5510, and the steel ball 5300 is received in the steel ball hole 5510. The steel ball hole 5510 in the fixed sleeve 5500 stabilizes the steel ball 5300 and prevents sliding misalignment that could interfere with rotation of the outer cutter tube 120.

The power handle of the present embodiment further comprises a speed reducer 5600. A proximal end of the inner cutter tube 110 is connected to the speed reducer 5600, and a proximal end of the outer cutter tube 120 is disposed outside the speed reducer 5600. As used herein, "proximal" refers to an end away from the cutter head that planes bone tissue. The speed reducer 5600 of the present embodiment may be a conventional speed reducer.

The power handle of the present embodiment further comprises a front housing 5710 and a rear housing 5720 that are connected to form an outer housing of the power handle. The outer housing is connected to both the handwheel 5210 and the fixed sleeve 5500.

A hollow interior of the inner cutter tube 110 forms a liquid suction passage 5800, and a gap between the inner cutter tube 110 and the outer cutter tube 120 forms a water injection passage 5900. Fluid may be injected to the cutter head through the water injection passage 5900 and discharged from the cutter head to cool and flush a planing site of the cutter head assembly, and waste fluid may be aspirated from the cutter head through the suction passage 5800.

When rotation of the outer cutter tube 120 is needed, the handwheel 5210 is rotated until a portion of the steel ball 5300 is received in the steel ball relief groove 5211. Further rotation of the handwheel 5210 causes the retaining protrusion 5213 to slide into abutment with the inner wall of the retaining notch 52211, such that the handwheel 5210 drives the retaining sleeve 5220 to rotate. The retaining sleeve 5220 drives the steering sleeve 5230 to rotate, and the steering sleeve 5230 drives the outer cutter tube 120 to rotate. Upon rotation of the steering sleeve 5230 until the steel ball 5300 snaps into another steel ball groove 5231, the handwheel 5210 is released. The handwheel 5210 returns rotationally in an opposite direction under torque of the torsion spring 5400. The ball-pressing protrusion 5212 returned to the inner wall of the handwheel 5210 presses the steel ball 5300 firmly into the other steel ball groove 5231, thereby locking the outer cutter tube 120. The fixed sleeve remains stationary throughout rotation of the handwheel.

In the present embodiment, the fixed sleeve of the power handle maintains the position of the steel ball 5300 via the steel ball hole 5510, preventing free sliding of the steel ball 5300 within the handwheel 5210. During rotation of the steering sleeve 5230 driven by the handwheel 5210, rotational damping is provided, which cooperates with the steel ball grooves 5231 on the steering sleeve 5230 to provide a detent feel for rotation of the outer cutter tube 120. The retaining ring 5520 inside the fixed sleeve 5500 and the slotted ring 5232 on the steering sleeve 5230 contact and abut each other, preventing axial sliding of the steering sleeve 5230 and stabilizing rotational motion of the outer cutter tube 120 relative to the inner cutter tube 110. When adjustment of a position of a planing window is needed, rotating the handwheel 5210 drives the outer cutter tube 120 to rotate relative to the inner cutter tube 110, providing highly convenient operation. In a locked state of the outer cutter tube 120, the steel ball 5300 is pressed firmly into a steel ball groove 5231 by the ball-pressing protrusion 5212, such that the outer cutter tube 120 cannot be rotated directly, ensuring safe and reliable use.

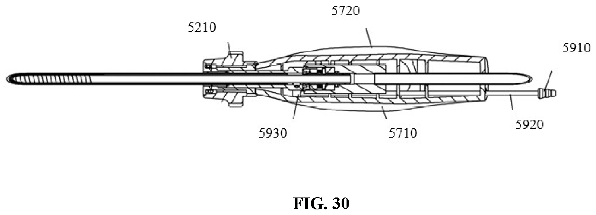

Referring to Figures 29 and 30, the present embodiment provides a planing cutter including the power handle and the cutter tube assembly of Embodiment 1, and further comprising a suction connector 5810, a suction tube 5820, a water injection connector 5910, and a water injection tube 5920. The suction connector 5810 communicates with the hollow interior of the inner cutter tube 110 via the suction tube 5820, and the water injection connector 5910 communicates with the water injection passage 5900 via the water injection tube 5920. An adapter sleeve 5610 is received over a proximal end of the cutter tube assembly. One end of the adapter sleeve 5610 is inserted into the fixed sleeve 5500, and an end of the adapter sleeve 5610 proximate the steel ball 5300 is provided with a seal 5620. The seal 5620 is received over the outer cutter tube 120. An end of the adapter sleeve 5610 distal from the steel ball 5300 is formed with a water injection port communicating with the water injection passage. A right-angle connector 5930 is connected to the water injection port and communicates with the water injection tube 5920. A proximal end of the outer cutter tube 120 is disposed in the adapter sleeve 5610, and a proximal end of the inner cutter tube 110 extends outwardly from the adapter sleeve 5610.

In the description of the present application, it will be understood that terms such as "upper," "lower," "front," "rear," "left," "right," "vertical," "lateral," "top," "bottom," "inner," and "outer" indicate orientations or positional relationships based on those shown in the drawings, are merely for convenience in describing the present application and simplifying the description, and do not indicate or imply that the referenced device or element must have a particular orientation or be constructed and operated in a particular orientation, and thus are not to be construed as limiting the present application.

Specific embodiments of the present invention have been described above. It will be understood that the present invention is not limited to the particular embodiments described above, and those skilled in the art may make various changes and modifications within the scope of the claims without affecting the essence of the present invention. The embodiments and features described in the present application may be combined with each other arbitrarily unless otherwise specified.

## Claims

1. A planing cutter, **characterized in** comprising:
a cutter tube assembly including an inner cutter tube and an outer cutter tube, wherein the inner cutter tube is rotatable relative to the outer cutter tube.

2. The planing cutter of claim 1, further comprising:
a housing, a power assembly, and a negative pressure suction assembly;
wherein the power assembly is disposed in the housing, one end of the cutter tube assembly extends into the housing, the outer cutter tube is fixedly connected to the housing, and the inner cutter tube is drivingly connected to an output end of the power assembly; and
one end of the negative pressure suction assembly extends into the housing and communicates with a cavity of the inner cutter tube, and the other end of the negative pressure suction assembly extends outwardly from the housing.

3. The planing cutter of claim 2, further comprising:
a water injection assembly;
wherein an end of the cutter tube assembly remote from the housing is formed with a water outlet, a water injection gap is defined between an inner wall of the outer cutter tube and an outer wall of the inner cutter tube, the water injection gap communicates with the water outlet, one end of the water injection assembly extends into the housing and communicates with the water injection gap between the outer cutter tube and the inner cutter tube, and the other end of the water injection assembly extends outwardly from the housing.

4. The planing cutter of claim 2, wherein the power assembly comprises a mounting base, a motor, and a gear transmission structure;
wherein the mounting base is fixedly mounted in the housing, the motor is seated on a lateral mounting portion of the mounting base, an output end of the motor abuts a vertical mounting portion of the mounting base, and an output shaft of the motor extends through the vertical mounting portion of the mounting base;
the gear transmission structure includes a driving gear, a dual transmission gear, and a driven gear; the driving gear is coaxially fixed to the output shaft of the motor, the dual transmission gear is rotatably mounted on the vertical mounting portion of the mounting base via a rotating shaft, the driven gear is drivingly connected to the inner cutter tube, and the dual transmission gear drivingly connects the driving gear and the driven gear.

5. The planing cutter of claim 4, wherein the negative pressure suction assembly comprises a negative pressure suction tube and a regulating valve;
an end of the inner cutter tube extending into the housing extends into the lateral mounting portion of the mounting base, one end of the negative pressure suction tube extends into the lateral mounting portion of the mounting base, and the other end of the negative pressure suction tube extends outwardly from the housing;
the regulating valve is rotatably disposed on the lateral mounting portion of the mounting base and configured to connect or disconnect the negative pressure suction tube and the inner cutter tube, and a regulating portion of the regulating valve is located outside the housing;
seals are disposed at a connection between the inner cutter tube and the lateral mounting portion of the mounting base and at a rotational connection between the regulating valve and the lateral mounting portion of the mounting base.

6. The planing cutter of claim 5, wherein a limiting tube is sleeved on the negative pressure suction tube;
one end of the limiting tube abuts a rear end surface of the lateral mounting portion of the mounting base, and the other end of the limiting tube abuts a reinforcing rib inside the housing.

7. The planing cutter of claim 4, wherein a contact surface between the lateral mounting portion of the mounting base and the motor is a concave arcuate surface.

8. The planing cutter of claim 4, wherein the outer cutter tube is fixedly connected to a front end of the lateral mounting portion of the mounting base via a fixed connection sleeve;
the water injection assembly comprises a water injection tube, one end of the water injection tube is disposed inside the housing, the other end of the water injection tube extends outwardly from the housing, the fixed connection sleeve is formed with a water injection connection hole, an end of the water injection tube inside the housing communicates with the water injection connection hole, and the water injection connection hole communicates with the water injection gap between the outer cutter tube and the inner cutter tube;
a seal and a bearing are disposed between an end of the fixed connection sleeve proximate to the mounting base and the inner cutter tube.

9. A drill bit structure for planing, wherein one end of an inner cutter tube is coaxially fixed with a drill bit, the drill bit is rotatable relative to an outer cutter tube, and an inner bore of the drill bit communicates with an inner passage of the inner cutter tube;
the drill bit is formed with radial suction bores evenly distributed along a circumference of the drill bit, and each of the radial suction bores communicates with the inner bore of the drill bit.

10. A planing drill bit assembly, **characterized in** comprising:
a cutter tube assembly including an inner cutter tube and an outer cutter tube, wherein the inner cutter tube is rotatable relative to the outer cutter tube;
wherein the outer cutter tube includes a double-layer wall, a water injection gap is defined between the double-layer wall of the outer cutter tube, a drill bit connection cylinder is formed with a water outlet hole, and the water outlet hole communicates with the water injection gap between the double-layer wall;
a rotating drill bit is formed with a suction bore in communication with an inner cavity of the inner cutter tube.

11. The planing drill bit assembly of claim 10, further comprising:
the rotating drill bit, the drill bit connection cylinder, and a power adapter sleeve;
wherein one end of the drill bit connection cylinder extends into the outer cutter tube and is fixedly connected thereto, one end of the rotating drill bit extends through an interior of the drill bit connection cylinder and into the inner cutter tube, the rotating drill bit is coaxially fixed to the inner cutter tube via the power adapter sleeve, and the rotating drill bit is rotatably engaged with the drill bit connection cylinder;
the power adapter sleeve abuts and limits one end of the drill bit connection cylinder, and the other end of the rotating drill bit is formed with an abutment surface that abuts and limits the other end of the drill bit connection cylinder.

12. A planing cutter head for a planing cutter, wherein one end of an outer cutter tube is formed with an outer planing window, the outer planing window is formed with outer planing teeth, the outer planing teeth include a plurality of continuous tooth portions, and each tooth portion of the outer planing teeth is inclined outwardly;
one end of an inner cutter tube is formed with an inner planing window, the inner planing window is formed with inner planing teeth, the inner planing teeth include a plurality of continuous tooth portions, and each tooth portion of the inner planing teeth is inclined inwardly.

13. A planing cutter head for a planing cutter, wherein one end of an outer cutter tube is formed with an outer planing window, and a continuous smooth cutting edge is formed at a periphery of an opening of the outer planing window;
one end of an inner cutter tube is formed with an inner planing window, two axially opposite sides of the inner planing window are each formed with one tooth portion, the two tooth portions are arranged opposite each other, and a smooth cutting edge is formed at a periphery of an opening of the inner planing window.

14. A rotary drive handle configured to be sleeved on a cutter tube assembly, the cutter tube assembly including an inner cutter tube and an outer cutter tube sleeved on the inner cutter tube, the rotary drive handle comprising:
a drive assembly configured to drive the outer cutter tube to rotate relative to the inner cutter tube, the drive assembly including a drive sleeve and a handwheel, the drive sleeve being sleeved on the outer cutter tube and configured to rotate the outer cutter tube, the handwheel being sleeved on the drive sleeve and configured to rotate the drive sleeve;
a limiting assembly configured to restrict rotation of the drive sleeve and provide a detent feel for rotation of the outer cutter tube, the limiting assembly being fixedly sleeved on the cutter tube assembly, one end of the limiting assembly being inserted into the handwheel and engaged with the drive sleeve;
an elastic member configured to drive the handwheel to rotate and return, mounted between the drive sleeve and the handwheel.

15. The rotary drive handle of claim 14, wherein the limiting assembly includes an adapter sleeve, a plurality of limiting grooves are evenly distributed along a circumference of an outer wall of the adapter sleeve, the drive sleeve is formed with an elastic limiting cylinder matching the limiting grooves, a limiting protrusion is disposed inside the handwheel, and when the drive sleeve is in a locked state, the limiting cylinder is engaged in one of the limiting grooves and the limiting protrusion contacts and abuts the limiting cylinder;
an elastic member mounting groove and a protrusion sliding groove are formed on an outer wall of the drive sleeve, the elastic member is disposed in the elastic member mounting groove, and the limiting protrusion is slidably disposed in the protrusion sliding groove;
a front limiting sleeve is mounted between the drive sleeve and the cutter tube assembly, the front limiting sleeve is sleeved on the cutter tube assembly, a bushing is disposed inside the drive sleeve, a rear limiting sleeve is mounted between the limiting assembly and the cutter tube assembly, the rear limiting sleeve is sleeved on the cutter tube assembly, a fixed sleeve is mounted between the rear limiting sleeve and the front limiting sleeve, and the fixed sleeve is sleeved on the cutter tube assembly;
a proximal end of the outer cutter tube is disposed inside the limiting assembly, and the inner cutter tube extends through the limiting assembly and is connected to an external power mechanism.

16. A cutter head steering structure configured to be sleeved on a cutter tube assembly, the cutter tube assembly including an inner cutter tube and an outer cutter tube sleeved on the inner cutter tube, wherein the cutter head steering structure comprises:
a handwheel, a fixed sleeve, a drive sleeve assembly, and a steel ball;
wherein the drive sleeve assembly is fixedly sleeved on the outer cutter tube and configured to drive the outer cutter tube to rotate relative to the inner cutter tube;
the handwheel is drivingly connected to the drive sleeve assembly, rotation of the handwheel drives the drive sleeve assembly to rotate, and the handwheel is sleeved on the fixed sleeve;
the steel ball is disposed inside the handwheel and configured to restrict rotation of the drive sleeve assembly;
the fixed sleeve is fixedly disposed and configured to maintain a position of the steel ball and restrict axial sliding of the drive sleeve assembly, and a spring is disposed between the handwheel and the fixed sleeve and configured to drive the handwheel to slide and return.

17. The cutter head steering structure of claim 16, wherein the drive sleeve assembly includes a directional sleeve and a retaining ring, the retaining ring is fixedly sleeved on the directional sleeve, rotation of the retaining ring drives the directional sleeve to rotate, the directional sleeve is sleeved on the outer cutter tube, and rotation of the directional sleeve drives the outer cutter tube to rotate;
a plurality of arc-shaped grooves are evenly distributed along a circumference of an outer wall of one end of the directional sleeve, a tapered cavity is defined inside the handwheel, and when the outer cutter tube is in a locked state, one end of the steel ball is disposed in one of the arc-shaped grooves and an inner wall of the tapered cavity contacts and abuts the steel ball;
a mounting ring is fixedly sleeved on an outer wall of one end of the directional sleeve, the plurality of arc-shaped grooves are formed on the mounting ring, an end of the fixed sleeve proximate to the directional sleeve is formed with a snap ring, the mounting ring is disposed inside the fixed sleeve, and the snap ring contacts and abuts one end of the mounting ring;
a clamping groove is formed on an outer side wall of the retaining ring, a through hole is formed in the handwheel at a position corresponding to the clamping groove, and a pin shaft is inserted into the through hole and slidably disposed in the clamping groove;
a steel ball hole is formed in the fixed sleeve, and the steel ball is inserted into the steel ball hole;
an adapter sleeve is sleeved on the cutter tube assembly, a proximal end of the outer cutter tube is disposed in the adapter sleeve, a proximal end of the inner cutter tube extends outwardly from the adapter sleeve, the fixed sleeve is fixedly sleeved on the adapter sleeve, and one end surface of the adapter sleeve contacts and abuts the drive sleeve assembly.

18. A power handle configured to be sleeved on a cutter tube assembly, the cutter tube assembly including an inner cutter tube and an outer cutter tube sleeved on the inner cutter tube, the power handle comprising:
a drive assembly configured to drive the outer cutter tube to rotate relative to the inner cutter tube, the drive assembly including a handwheel, a retaining sleeve, and a steering sleeve, the steering sleeve being sleeved on the outer cutter tube and configured to rotate the outer cutter tube, the retaining sleeve being sleeved on the steering sleeve and configured to rotate the steering sleeve, the handwheel being sleeved on the retaining sleeve and configured to rotate the retaining sleeve;
a steel ball configured to restrict rotation of the steering sleeve;
a torsion spring configured to drive the handwheel to rotate and return, disposed between the retaining sleeve and the handwheel;
a fixed sleeve configured to maintain a position of the steel ball and restrict axial sliding of the steering sleeve, fixedly mounted inside the handwheel.

19. The power handle of claim 18, wherein a plurality of steel ball grooves are evenly distributed along a circumference of an outer wall of the steering sleeve, a steel ball relief groove is formed on an inner wall of the handwheel, and both the steel ball grooves and the steel ball relief groove are configured to receive the steel ball;
a ball-pressing protrusion is disposed on an inner wall of the handwheel and configured to press the steel ball tightly, and the ball-pressing protrusion is disposed adjacent to the steel ball relief groove;
the retaining sleeve includes a baffle and a connecting tube that are coaxially fixed to each other, a limiting notch is formed in the baffle, a limiting protrusion is disposed on an inner wall of the handwheel and slidably disposed in the limiting notch, the connecting tube is disposed inside the handwheel, and the torsion spring is sleeved on the connecting tube;
a limiting groove is formed in the connecting tube, a limiting post is disposed on the steering sleeve at a position corresponding to the limiting groove and inserted into the limiting groove;
a slotted ring is disposed on the steering sleeve, the steel ball groove is formed in the slotted ring, the slotted ring is disposed inside the fixed sleeve, a limiting ring is disposed inside one end of the fixed sleeve, and one end of the limiting ring contacts and abuts one end of the slotted ring;
a steel ball hole is formed in the fixed sleeve, and the steel ball is inserted into the steel ball hole;
the power handle further comprises a speed reducer, a proximal end of the inner cutter tube is connected to the speed reducer, a proximal end of the outer cutter tube is disposed outside the speed reducer, a hollow interior of the inner cutter tube forms a liquid suction passageway, and a gap between the inner cutter tube and the outer cutter tube forms a water injection passageway.
